(19) Europäisches Patentamt / European Patent Office / Office européen des brevets

(11) **EP 3 254 706 B1**

(12) **EUROPEAN PATENT SPECIFICATION**

(45) Date of publication and mention
of the grant of the patent:
**27.01.2021 Bulletin 2021/04**

(21) Application number: **16746329.8**

(22) Date of filing: **04.02.2016**

(51) Int Cl.:
*A61L 9/01* $^{(2006.01)}$     *B01J 20/24* $^{(2006.01)}$
*B01J 20/30* $^{(2006.01)}$     *D06M 11/83* $^{(2006.01)}$
*C08L 1/04* $^{(2006.01)}$

(86) International application number:
**PCT/JP2016/000588**

(87) International publication number:
**WO 2016/125498 (11.08.2016 Gazette 2016/32)**

(54) **DEODORANT AND METHOD FOR MANUFACTURING SAME**

DEODORANT UND VERFAHREN ZUR HERSTELLUNG DAVON

DÉODORANT ET PROCÉDÉ DE FABRICATION DE CELUI-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.02.2015 JP 2015020426
04.02.2015 JP 2015020429**

(43) Date of publication of application:
**13.12.2017 Bulletin 2017/50**

(73) Proprietor: **Zeon Corporation
Tokyo 100-8246 (JP)**

(72) Inventor: **SONE, Atsushi
Tokyo 100-8246 (JP)**

(74) Representative: **Maiwald Patent- und
Rechtsanwaltsgesellschaft mbH
Elisenhof
Elisenstraße 3
80335 München (DE)**

(56) References cited:
**EP-A1- 2 395 027    EP-A1- 2 816 646
EP-A1- 3 255 064    WO-A1-2015/170613
JP-A- S62 138 538    JP-A- 2003 339 836
JP-A- 2013 104 133    JP-A- 2014 070 158**

Remarks:
The file contains technical information submitted after
the application was filed and not included in this
specification

**Description**

TECHNICAL FIELD

The present disclosure relates to deodorants having good deodorant properties which include metal-containing oxidized cellulose nanofibers, and methods of producing the same.

BACKGROUND

Various studies have heretofore been made on using cellulose fibers as a deodorant. For example, PTL 1 discloses a technique of imparting deodorizing properties to cellulose fibers of fabrics by copper or zinc carboxymethylation of the cellulose fibers.

Further, PTL 2 discloses an odor-absorbing cellulose-based deodorant sheet which has a deodorizing effect particularly on such odors as hydrogen sulfide and garlic odor. The deodorant sheet has a layer in which a micronized powder of cellulose fibers modified to have carboxyl groups substituted with metal ions such as copper or zinc ions (hereinafter occasionally referred to as "metal-substituted carboxyl groups") is added in a cellulose pulp.

Cellulose fibers containing or supporting a metal are also known from PTL 3 and PTL 4.

PTL 3 discloses antibacterial fine cellulose characterized in that metal microparticles made of one or more kind of metal or a chemical compound thereof are supported on the surface of fine cellulose having a carboxyl group at least on the crystal surface.

PTL 4 on the other hand is directed to a suspension of cellulose fibers suitably used for producing a gas barrier film for controlling permeation of various gases such as oxygen, water vapor, carbon dioxide, nitrogen, and limonene, a method for producing the same, and a molded article using the same, wherein the cellulose fiber contains a cellulose fiber having an average fiber diameter of not more than 200 nm, the content of carboxyl groups in cellulose composing the cellulose fibers is 0.1 to 2 mmol/g, and a metal selected from a polyvalent metal and a monovalent metal is contained in the cellulose fiber to form a counter ion for the carboxyl group, provided that the counter ion of sodium only is excluded.

CITATION LIST

Patent Literature

PTL 1: JP2002-371462A

PTL 2: JPH11-315499A

PTL 3: JP2014-70158 A

PTL 4: EP 2 395 027 A1

SUMMARY

(Technical Problem)

Deodorization of odors generated in living or other environments has been required more strictly than ever before due to increasing environmental consciousness.

However, the technique disclosed in PTL 1 is still unsatisfactory with regard to deodorizing properties because the dyeability of fabrics containing cellulose fibers is taken into account.

Even the technique disclosed in PTL 2 is still unsatisfactory with regard to deodorant properties per unit amount of the deodorant sheet used.

Specifically, with the technique disclosed in PTL 1, only the cellulose-based fibers present on the back side of cellulose fiber fabric are metal carboxymethylated to ensure dyeability and therefore the amount of metal-carboxymethylated cellulose fibers is too small to attain a sufficient level of deodorizing properties.

Further, with the technique disclosed in PTL 2, metal-substituted carboxyl groups are first introduced to a bulk bleached pulp and then the bleached pulp is disintegrated into individual modified cellulose fibers. Hence, each strand of modified cellulose fibers cannot have a sufficient number of metal-substituted carboxyl groups introduced, resulting in failure to attain a sufficient level of deodorizing properties.

(Solution to Problem)

It would therefore be helpful to provide a deodorant that effectively deodorizes odors generated in living or other environments, as well as an advantageous method of producing the deodorant.

The inventors made extensive studies to achieve the forgoing object. The inventors conceived of a new idea of imparting a high deodorant effect to individual strands of oxidized cellulose fibers by allowing well-dispersed oxidized cellulose fibers to contain a metal other than sodium, rather than by introducing a metal such as copper to cellulose fibers in fabric or pulp to impart a deodorant effect.

The inventors then made further studies and established that: oxidation of native cellulose in the presence of an oxidation catalyst such as N-oxyl compound and subsequent mechanical dispersing treatment of the resulting oxidized cellulose results in a dispersion in which highly crystalline ultrafine fibers (oxidized cellulose nanofibers) with diameters of 100 nm or less are well dispersed in a dispersion medium such as water; and that substituting a metal, derived from the oxidizing agent or the like and contained in the oxidized cellulose nanofibers in salt form, by a metal other than sodium results in oxidized cellulose nanofibers that exhibit superior deodorizing properties. The inventors completed the present disclosure based on these findings.

Specifically, the gist of the present invention is as follows:

1. A deodorant including:

metal-containing oxidized cellulose nanofibers containing a metal other than sodium in salt form and having a number-average fiber diameter of 100 nm or less, wherein
the metal-containing oxidized cellulose nanofibers have an average degree of polymerization of 100 to 2,000, wherein the metal other than sodium is at least one metal selected from the group consisting of copper, zinc, and silver,
wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and
wherein the metal-containing oxidized cellulose nanofibers are metal-containing carboxylated cellulose nanofibers.

2. The deodorant of 1, wherein the metal-containing oxidized cellulose nanofibers have a number-average fiber length of 50 nm to 2,000 nm.

3. The deodorant of 1 or 2, further including a dispersion medium, wherein
the metal-containing oxidized cellulose nanofibers are dispersed in the
dispersion medium.

4. The deodorant of 3, wherein the dispersion medium is water.

5. A method of producing a deodorant, including:

contacting oxidized cellulose nanofibers containing a first metal in salt form, dispersed in a solvent, with a salt of a second metal other than the first metal to produce metal-containing oxidized cellulose nanofibers containing the second metal in salt form and having a number-average fiber diameter of 100 nm or less, wherein
the metal-containing oxidized cellulose nanofibers have an average degree of polymerization of 100 to 2,000, wherein the first metal is sodium and the second metal is at least one metal selected from the group consisting of copper, zinc, and silver,
wherein the average degree of polymerization is measured as with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and
wherein the oxidized cellulose nanofibers are carboxylated cellulose nanofiber.

6. A method of producing a deodorant, including:

contacting oxidized cellulose nanofibers containing a first metal in salt form, dispersed in a solvent, with a strong acid to substitute ions of the first metal contained in salt form by hydrogen atoms; and
contacting the oxidized cellulose nanofibers in which the ions of the first metal have been substituted by hydrogen atoms, dispersed in a solvent, with a salt of a second metal other than the first metal to produce metal-containing oxidized cellulose nanofibers containing the second metal in salt form and having a number-average fiber diameter of 100 nm or less, wherein

the metal-containing oxidized cellulose nanofibers have an average degree of polymerization of 100 to 2,000, wherein the first metal is sodium and the second metal is at least one metal selected from the group consisting of copper, zinc, and silver,

wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and

wherein the oxidized cellulose nanofibers are carboxylated cellulose nanofibers.

7. The method of producing a deodorant of 5 or 6, wherein the metal-containing oxidized cellulose nanofibers have a number-average fiber length of 50 nm to 2,000 nm.

8. The method of producing a deodorant of any one of 5 to 7, further including dispersing the metal-containing oxidized cellulose nanofibers in a dispersion medium.

9. The method of producing a deodorant of 8, wherein the dispersion medium is water.

(Advantageous Effect)

According to the present disclosure, it is possible to provide a deodorant that effectively deodorizes odors generated in living or other environments.

DETAILED DESCRIPTION

The present disclosure will be described in detail below.

The disclosed deodorant is used for deodorizing (or killing) odors generated in living or other environments, and comprises metal-containing oxidized cellulose nanofibers containing a metal other than sodium in salt form and having a number-average fiber diameter of 100 nm or less, wherein the metal-containing oxidized cellulose nanofibers have an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers. The disclosed deodorant can be produced using the disclosed method of producing a deodorant. The following describes the disclosed method of producing a deodorant, and the disclosed deodorant which may be produced using the disclosed method.

The disclosed deodorant can be used for any purpose, e.g., deodorization of, for example, ammonia, methyl mercaptan or hydrogen sulfide.

(Method of Producing Deodorant)

An example of the disclosed method of producing a deodorant is a method of producing a deodorant which comprises metal-containing oxidized cellulose nanofibers containing a metal other than sodium in salt form and having a number-average fiber diameter of 100 nm or less, wherein the metal-containing oxidized cellulose nanofibers have an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers.

In this exemplary production method, using oxidized cellulose nanofibers containing a first metal in salt form as a raw material, either one of the following methods (i) and (ii) is used to substitute ions of the first metal in the oxidized cellulose nanofibers by ions of a second metal to produce metal-containing oxidized cellulose nanofibers containing the second metal in salt form and having a number-average fiber diameter of 100 nm or less, wherein the metal-containing oxidized cellulose nanofibers have an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers. As used herein, the term "second metal" means a metal other than the first metal.

Method (i): oxidized cellulose nanofibers containing a first metal in salt form, dispersed in a solvent, are contacted with a salt of a second metal (first production method); and

Method (ii): oxidized cellulose nanofibers containing a first metal in salt form, dispersed in a solvent, are contacted with a strong acid to substitute ions of the first metal contained in salt form by hydrogen atoms, after which the oxidized cellulose nanofibers in which the ions of the first metal have been substituted by hydrogen atoms, dispersed in a solvent, are contacted with a salt of a second metal (second production method).

<First Production Method>

In the first production method described above, oxidized cellulose nanofibers containing a first metal in salt form, dispersed in a solvent, are contacted with a salt of a second metal to substitute at least some, preferably all, of the ions of the first metal of the oxidized cellulose nanofibers by ions of the second metal (metal substitution step).

Optionally, the metal-containing oxidized cellulose nanofibers containing the second metal in salt form obtained from the metal substitution step are then washed (washing step) and, where necessary, further dispersed in a dispersion medium (dispersing step) to afford metal-containing oxidized cellulose nanofibers containing the second metal in salt form and having a number-average fiber diameter of 100 nm or less, wherein the metal-containing oxidized cellulose nanofibers have an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers.

[Metal Substitution Step]

Any oxidized cellulose nanofibers is disclosed herein as long as they are obtainable by oxidation of cellulose and contain the first metal in salt form, e.g., oxidized cellulose nanofibers disclosed in WO2011/074301 can be used. In the present invention, carboxylated cellulose nanofibers containing the first metal in salt form are used. The use of carboxylated cellulose nanofibers results in metal-containing oxidized cellulose nanofibers having superior dispersibility.

Any carboxylated cellulose nanofibers containing the first metal in salt form can be used. For example, carboxylated cellulose nanofibers in which the primary hydroxyl groups at C6 of the $\beta$-glucose units (building blocks of cellulose) are selectively oxidized can be used. Examples of methods of selectively oxidizing the primary hydroxyl groups at C6 of the $\beta$-glucose units include oxidation methods that use N-oxyl compounds as an oxidation catalyst, such as TEMPO-catalyzed oxidation described below.

In TEMPO-catalyzed oxidation, native cellulose as a raw material is oxidized in an aqueous medium by the action of an oxidizing agent using TEMPO (2,2,6,6-tetramethyl-1-piperidine-N-oxyl) or a derivative thereof as an oxidation catalyst.

The native cellulose subjected to the oxidation treatment is then optionally washed and then dispersed in an aqueous medium such as water to afford an aqueous dispersion of cellulose nanofibers having a number-average fiber diameter of 100 nm or less, preferably 10 nm or less, and having a group in the form of carboxylate (carboxylated cellulose nanofibers), wherein the metal-containing oxidized cellulose nanofibers have an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers.

Native cellulose usable as the raw material can be purified cellulose isolated from cellulose biosynthesis system, such as a plant, animal or bacteria-producing gel.

Specific examples include cellulose isolated from coniferous wood pulp, deciduous wood pulp, cotton-based pulp such as cotton linter or cotton lint, non-wood-based pulp such as pulp from barley or bagasse pulp, bacterial cellulose, cellulose isolated from sea squirt, and cellulose isolated from sea grass.

From the perspective of increasing the efficiency of the oxidation reaction and thus the productivity of carboxylated cellulose nanofibers, isolated, purified native cellulose may be subjected to beating or other treatment to increase the surface area.

Further, it is preferred to use never-dried native cellulose that has been stored in a never-dried state after isolation and purification, since by doing so, bundles of microfibrils are kept in a state that allows for easy swelling, thereby improving the oxidation reaction efficiency and facilitating the production of carboxylated cellulose nanofibers with small fiber diameters.

TEMPO derivatives usable as oxidation catalysts include those having various functional groups at C4 of 2,2,6,6-tetramethyl-1-piperidine-N-oxyl (TEMPO). Examples of TEMPO derivatives include 4-acetamido-TEMPO, 4-carboxy-TEMPO, and 4-phosphonoxy-TEMPO. High reaction rate can be attained especially when TEMPO or 4-acetamido-TEMPO is used as the oxidation catalyst.

Examples of oxidizing agents include hypohalous acids or salts thereof (e.g., hypochlorous acid or salt thereof, hypobromous acid or salt thereof, and hypoiodous acid or salt thereof); halous acids or salts thereof (e.g., chlorous acid or salt thereof, bromous acid or salt thereof, and iodous acid or salt thereof); perhalogen acids or salts thereof (e.g., perchloric acid or salt thereof, and periodic acid or salt thereof); halogens (e.g., chlorine, bromine, and iodine); halogen oxides (e.g., $ClO$, $ClO_2$, $Cl_2O_6$, $BrO_2$, and $Br_3O_7$); nitrogen oxides (e.g., $NO$, $NO_2$, and $N_2O_3$); and peracids (e.g., hydrogen peroxide, peracetic acid, persulfuric acid, and perbenzoic acid). These oxidizing agents can be used alone or in combination, and also can be used in combination with oxidizing enzymes such as laccase.

Depending on the type of the oxidizing agent, bromide or iodide may be combined with the oxidizing agent for use as a co-oxidizing agent. For example, ammonium salts (ammonium bromide, ammonium iodide), alkali metal bromides or iodides, and alkaline earth metal bromides or iodides can be used. These bromides and iodides can be used alone or in combination.

When a metal salt has been used as the oxidizing agent during TEMPO-catalyzed oxidation, generally, the metal constituting the metal salt is contained in salt form in the resulting carboxylated cellulose nanofibers. That is, the metal constituting the metal salt is the first metal.

Among the oxidizing agents described above, it is preferred to use a sodium salt from the perspective of increasing the oxidation reaction rate, with sodium hypochlorite being more preferred, and a co-oxidizing agent of sodium hypochlorite and sodium bromide being particularly preferred. When a sodium salt has been used as the oxidizing agent, generally, carboxylated cellulose nanofibers containing sodium in salt form as the first metal are obtained.

Any condition and method known in the art used for TEMPO-catalyzed oxidation can be employed for the oxidation treatment. In the oxidation treatment, the primary hydroxyl groups at C6 of the β-glucose units are oxidized via aldehyde groups to carboxyl groups. From the perspective of imparting sufficient levels of desired characteristics to metal-containing oxidized cellulose nanofibers obtained from the raw material carboxylated cellulose nanofibers, the proportion of the primary hydroxyl groups oxidized to carboxyl groups is preferably 50mol% or more, more preferably 70mol% or more, even more preferably 90mol% or more.

Various dispersing devices (defibration devices) can be used for dispersing the carboxylated cellulose nanofibers after the oxidation treatment. Specifically, for example, a defibration device such as a household mixer, an ultrasonic homogenizer, a high-pressure homogenizer, a twin-screw kneader or a stone mill can be used. In addition, defibration devices that are commonly used for domestic use or industrial production can be used. In particular, defibration devices with a stronger beating power, such as various homogenizers and refiners, more efficiently provide an aqueous dispersion of carboxylated cellulose nanofibers with small fiber diameters.

It is preferred that the carboxylated cellulose nanofibers after the oxidation treatment are dispersed after increasing the purity by repeated cycles of washing with water and solid-liquid separation. In addition, when non-defibrated components remain in the aqueous dispersion after dispersing treatment, it is preferred to remove such non-defibrated components by centrifugation or other techniques.

In the metal substitution step, substitution of metal ions by a contact between oxidized cellulose nanofibers containing a first metal in salt form and a salt of a second metal can be accomplished by adding a solution or solid of the salt of the second metal to an aqueous dispersion of oxidized cellulose nanofibers obtained through the above-described TEMPO-catalyzed oxidation or other oxidation methods, and stirring the resulting mixture. In the metal substitution step, substitution of metal ions by contacting the salt of the second metal with the well-dispersed oxidized cellulose nanofibers in the manner as described above allows each individual strand of the oxidized cellulose nanofibers to effectively contain the second metal, resulting in metal-containing oxidized cellulose nanofibers having a superior deodorizing effect.

The salt of the second metal is a salt of a metal which conforms to characteristics desired to be imparted to the resulting metal-containing oxidized cellulose nanofibers. The first metal is sodium and the salt of the second metal is a salt of at least one metal selected from the group consisting of copper, zinc, and silver.

Metal-containing oxidized cellulose nanofibers obtained using a copper salt as the salt of the second metal (copper-containing oxidized cellulose nanofibers) are particularly superior in deodorizing hydrogen sulfide.

The second metal to be added to the dispersion of oxidized cellulose nanofibers can be in any salt form, such as halide, acetate, sulfate, or nitrate. In particular, from the perspective of improving the efficiency with which metal ions are substituted, the salt of the second metal is preferably a weak acid salt, more preferably an acetate.

Further, from the perspective of effective metal substitution on well-dispersed oxidized cellulose nanofibers to provide metal-containing oxidized cellulose nanofibers having a superior deodorizing effect, the solvent for the oxidized cellulose nanofibers containing the first metal in salt form is preferably water. The concentration of the oxidized cellulose nanofibers in the solvent is preferably 0.005% by mass or more, more preferably 0.01% by mass or more, even more preferably 0.05% by mass or more, and preferably 5% by mass or less, more preferably 3% by mass or less, even more preferably 2% by mass or less. If the concentration of the oxidized cellulose nanofibers is too low, it results in poor reaction efficiency and productivity. If the concentration of the oxidized cellulose nanofibers is too high, it results in high solvent viscosity making uniform stirring difficult to perform.

The time of stirring the mixture of the oxidized cellulose nanofibers and the salt of the second metal can be a time sufficient for effecting metal ion substitution, e.g., from 1 hour to 10 hours. Stirring temperature can for example range from 10°C to 50°C.

In the metal substitution step described above, when the oxidized cellulose nanofibers containing the first metal in salt form are contacted with the salt of the second metal in liquid, gelling of the oxidized cellulose nanofibers may occur. Even in such a case, performing the dispersing step after the optional washing step allows the resulting oxidized cellulose nanofibers to be well re-dispersed, so that metal-containing oxidized cellulose nanofibers having a number-average fiber diameter of 100 nm or less can be obtained.

[Washing Step]

In the optional washing step that follows the metal substitution step, the oxidized cellulose nanofibers after metal substitution are washed by any washing method known in the art, e.g., repeated cycles of centrifugation and replacement of supernatant with washing solution, or filtration and washing with a large quantity of washing solution.

Any washing solution can be used, e.g., water can be used. However, from the perspective of enhancing the efficiency of metal substitution of the oxidized cellulose nanofiber obtained from the metal substitution step, it is preferred to perform washing first using an aqueous solution of the salt of the second metal as washing solution, and then using water as washing solution.

[Dispersing Step]

In the dispersing step, the oxidized cellulose nanofibers containing the second metal in salt form are dispersed using a known dispersing device (defibration device) such as a household mixer, an ultrasonic homogenizer, a high-pressure homogenizer, a twin-screw kneader, or a stone mill. Non-defibrated components are removed where necessary by centrifugation or other techniques to provide a dispersion of metal-containing oxidized cellulose nanofibers.

In the dispersion obtained as described above, the metal-containing oxidized cellulose nanofibers are highly dispersed to an extent that the metal-containing oxidized cellulose nanofibers have a number-average fiber diameter of 100 nm or less, preferably 2 nm to 10 nm, more preferably 2 nm to 5 nm. Thus, with the dispersion, a deodorant is obtained that shows a superior deodorizing effect even when the amount used is small. Specifically, in addition to examples described later, for example, a dispersion containing the metal-containing oxidized cellulose nanofibers having a number-average fiber diameter of 100 nm or less can be used as it is as a spray deodorant which contains the metal-containing oxidized cellulose nanofibers as a deodorant component. Further, a dispersion containing the metal-containing oxidized cellulose nanofibers having a number-average fiber diameter of 100 nm or less may be either dried or formulated with other materials (e.g., polymers) for use as a deodorant formed of a shaped article containing the metal-containing oxidized cellulose nanofibers as a deodorant component. Further, fibers or paper may also be impregnated with the dispersion to produce a deodorant.

The metal-containing oxidized cellulose nanofibers obtained in the manner described above preferably have a number-average fiber length of 50 nm to 2,000 nm, more preferably 70 nm to 1,500 nm, even more preferably 100 nm to 1,000 nm, particularly preferably 400 nm to 600 nm. When the number-average fiber length is 50 nm or more, sufficiently high mechanical strength can be imparted to a shaped article as a deodorant formed using the metal-containing oxidized cellulose nanofibers. When the number-average fiber length is 2,000 nm or less, the dispersibility of the metal-containing oxidized cellulose nanofibers can be ensured, so that the dispersion can be sufficiently enriched with the metal-containing oxidized cellulose nanofibers.

The number-average fiber length of the metal-containing oxidized cellulose nanofibers can be adjusted for example by changing the number-average fiber length of the raw material native cellulose and the oxidizing treatment conditions, the condition used for dispersing (defibrating) the carboxylated cellulose nanofibers after the oxidation treatment, and/or the condition used for dispersing (defibrating) after the metal substitution step. Specifically, by prolonging the time of dispersing treatment (defibrating treatment), the number-average fiber length can be reduced.

The metal-containing oxidized cellulose nanofibers have an average degree of polymerization (average number of glucose units in the cellulose molecules) of 100 to 2,000, preferably 300 to 1,500, more preferably 500 to 1,000, and particularly preferably 500 to 700. When the average degree of polymerization is 100 or more, sufficiently high mechanical strength can be imparted to a shaped article as a deodorant formed using the metal-containing oxidized cellulose nanofibers. When the average degree of polymerization is 2,000 or less, the dispersibility of the metal-containing oxidized cellulose nanofibers can be ensured, so that the dispersion can be sufficiently enriched with the metal-containing oxidized cellulose nanofibers.

The average degree of polymerization of the metal-containing oxidized cellulose nanofibers can be adjusted for example by changing the average degree of polymerization of the raw material native cellulose and the oxidizing treatment conditions, the condition used for dispersing (defibrating) the carboxylated cellulose nanofibers after the oxidation treatment, and/or the condition used for dispersing (defibrating) after the metal substitution step.

<Second Production Method>

In the second production method, first, oxidized cellulose nanofibers containing a first metal in salt form, dispersed in a solvent, are contacted with a strong acid to substitute at least some, preferably all, of the ions of the first metal of the oxidized cellulose nanofibers by hydrogen atoms (hydrogen substitution step). Next, the oxidized cellulose nanofibers obtained from the above hydrogen substitution step are optionally washed (first washing step) and further dispersed in a dispersion medium (first dispersing step). Subsequently, the oxidized cellulose nanofibers in which the ions of the first metal have been substituted by hydrogen atoms, dispersed in a solvent, are contacted with a salt of a second metal to substitute at least some, preferably all of, the hydrogen atoms introduced in the hydrogen substitution step and the ions of the first metal which have not been substituted by hydrogen atoms (metal substitution step). Thereafter, optionally, the metal-containing oxidized cellulose nanofibers containing the second metal in salt form, obtained from the metal substitution step, are washed (second washing step) and, where necessary, further dispersed in a dispersion medium (second dispersing step) to afford metal-containing oxidized cellulose nanofibers containing the second metal in salt form and having a number-average fiber diameter of 100 nm or less, wherein the metal-containing oxidized cellulose nanofibers have an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers.

In this second production method, since the hydrogen substitution step precedes the metal substitution step, it is possible to increase the substitution of the first metal by the second metal compared to the first production method described above where the first metal is directly substituted by the second metal.

[Hydrogen Substitution Step]

The oxidized cellulose nanofibers containing the first metal in salt form in the hydrogen substitution step can be the oxidized cellulose nanofibers used in the above-described first production method.

In the hydrogen substitution step, substitution of ions of the first metal by hydrogen atoms by a contact between the oxidized cellulose nanofibers containing the first metal in salt form and a strong acid can be accomplished by adding a solution of a strong acid to a dispersion of oxidized cellulose nanofibers obtained by TEMPO-catalyzed oxidation or other oxidation methods, and stirring the resulting mixture.

Any strong acid can be used as long as it is capable of substituting ions of the first metal by hydrogen atoms (i.e., substituting the carboxyl groups of the oxidized cellulose nanofibers by carboxylic acid form). For example, it is possible to use hydrochloric acid, sulfuric acid or nitric acid, with hydrochloric acid being preferred.

The time of stirring the mixture of the oxidized cellulose nanofibers and strong acid can be a time sufficient for effecting substitution of metal ions by hydrogen atoms, e.g., from 10 minutes to 5 hours. Stirring temperature can for example range from 10°C to 50°C.

[First Washing Step]

In the optional first washing step that follows the hydrogen substitution step, the hydrogen-substituted oxidized cellulose nanofibers are washed to remove strong acid by any washing method known in the art, e.g., repeated cycles of centrifugation and replacement of supernatant with washing solution, or filtration and washing with a large quantity of washing solution. In this way, by carrying out the first washing step, it is possible to remove strong acid and to prevent carboxyl groups of carboxylic acid form from remaining in the metal substitution step described later. As a result, in the metal substitution step, the hydrogen atoms introduced in the hydrogen substitution step and the ions of the first metal which have not been substituted by hydrogen atoms can be sufficiently substituted by ions of the second metal.

Any washing solution can be used in the first washing step, e.g., water can be used. However, from the perspective of enhancing the efficiency with which the carboxyl groups of the oxidized cellulose nanofibers are substituted by carboxylic acid form, it is preferred to perform washing first using a solution of strong acid as washing solution, and then using water as washing solution.

[First Dispersing Step]

In the first dispersing step, the oxidized cellulose nanofibers in which carboxyl groups are substituted by carboxylic acid form are dispersed in a dispersion medium such as water to afford a dispersion of oxidized cellulose nanofibers in which ions of the first metal are substituted by hydrogen atoms. In the first dispersing step, the oxidized cellulose nanofibers in which carboxyl groups are substituted by carboxylic acid form need not be completely dispersed in the dispersion medium using a known dispersing device (defibrating device) or the like.

[Metal Substitution Step]

The metal substitution step of the second production method can be performed in the same way as that of the first production method except that oxidized cellulose nanofibers in which ions of the first metal are substituted by hydrogen atoms are contacted with a salt of a second metal. A preferred mode of the metal substitution step of the second production method is also the same as that of the metal substitution step of the first production method.

[Second Washing Step and Second Dispersing Step]

The second washing step and the second dispersing step in the second production method can also be performed in the same way as those of the first production method described above. Further, preferred modes of the second washing step and the second dispersing step of the second production method are also the same as those of the washing step and the dispersing step of the first production method.

In the dispersion obtained as described above, the metal-containing oxidized cellulose nanofibers containing the second metal in salt form are highly dispersed to an extent that the metal-containing oxidized cellulose nanofibers have a number-average fiber diameter of 100 nm or less, preferably 2 nm to 10 nm, more preferably 2 nm to 5 nm. Thus, with the dispersion, a deodorant is obtained that shows a superior deodorizing effect even when the amount used is small. Specifically, in addition to examples described later, for example, a dispersion containing the metal-containing oxidized cellulose nanofibers having a number-average fiber diameter of 100 nm or less and an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers can be used as it is as a spray deodorant which contains the metal-containing oxidized cellulose nanofibers as a deodorant component. Alternatively, a dispersion containing the metal-containing oxidized cellulose nanofibers having a number-average fiber diameter of 100 nm or less and an average degree of polymerization of 100 to 2,000 , wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers may be either dried or formulated with other materials (e.g., polymers) for use as a deodorant formed of a shaped article containing the metal-containing oxidized cellulose nanofibers as a deodorant component. Further, fibers or paper may also be impregnated with the dispersion to produce a deodorant.

The metal-containing oxidized cellulose nanofibers containing the second metal in salt form obtained as described above preferably have a number-average fiber length of 50 nm to 2,000 nm, more preferably 70 nm to 1,500 nm, even more preferably 100 nm to 1,000 nm, particularly preferably 400 nm to 600 nm.

When the number-average fiber length is 50 nm or more, sufficiently high mechanical strength can be imparted to a shaped article as a deodorant formed using the metal-containing oxidized cellulose nanofibers. When the number-average fiber length is 2,000 nm or less, the dispersibility of the metal-containing oxidized cellulose nanofibers can be ensured, so that the dispersion can be sufficiently enriched with the metal-containing oxidized cellulose nanofibers.

The number-average fiber length of the metal-containing oxidized cellulose nanofibers containing the second metal in salt form can be adjusted for example by changing the number-average fiber length of the raw material native cellulose and the oxidizing treatment conditions, the condition used for dispersing (defibrating) the carboxylated cellulose nanofibers after the oxidation treatment, and/or the condition used for dispersing (defibrating) the oxidized cellulose nanofibers containing the second metal in salt form after the metal substitution step. Specifically, by prolonging the time for dispersing treatment (defibrating treatment), the number-average fiber length can be reduced.

The metal-containing oxidized cellulose nanofibers containing the second metal in salt form have an average degree of polymerization (average number of glucose units in the cellulose molecules) of 100 to 2,000, preferably 300 to 1,500, more preferably 500 to 1,000, and particularly preferably 500 to 700. When the average degree of polymerization is 100 or more, sufficiently high mechanical strength can be imparted to a shaped article as a deodorant formed using the metal-containing oxidized cellulose nanofibers. When the average degree of polymerization is 2,000 or less, the dispersibility of the metal-containing oxidized cellulose nanofibers can be ensured, so that the dispersion can be sufficiently enriched with the metal-containing oxidized cellulose nanofibers.

The average degree of polymerization of the metal-containing oxidized cellulose nanofibers can be adjusted for example by changing the average degree of polymerization of the raw material native cellulose and the oxidizing treatment conditions, the condition used for dispersing (defibrating) the carboxylated cellulose nanofibers after the oxidation treatment, and/or the condition used for dispersing (defibrating) after the metal substitution step.

(Deodorant)

A deodorant which may be produced using the above-described production method comprises metal-containing oxidized cellulose nanofibers having a number-average fiber diameter of 100 nm or less and an average degree of polymerization of 100 to 2,000, which exhibit superior deodorant properties.

Specific examples of deodorants which may be produced using the above-described production method include: spray deodorants in which metal-containing oxidized cellulose nanofibers having a number-average fiber diameter of 100 nm or less, preferably 2 nm to 10 nm, more preferably 2 nm to 5 nm and an average degree of polymerization of 100 to 2,000, wherein the first metal is sodium and the second metal is at least one metal selected from the group consisting of copper, zinc, and silver, wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and wherein the oxidized cellulose nanofibers are carboxylated cellulose nanofibers, are dispersed in a dispersion medium such as water; deodorants formed of an aggregate (e.g., membrane or non-woven fabric) of metal-containing oxidized cellulose nanofibers having the above-described number-average fiber diameter and average degree of polymerization; deodorants obtained by coating shaped articles with a dispersion of oxidized cellulose nanofibers having the above-described number-average fiber diameter and average degree of polymerization dispersed in a dispersion medium such as water, and drying the dispersion; deodorants formed of shaped articles obtained by shaping of composite materials of metal-containing oxidized cellulose nanofibers having the above-described number-average fiber diameter and average degree of polymerization and polymers or other materials; and deodorants obtained by impregnating fibers, paper or the like with a dispersion of oxidized cellulose nanofibers having the above-described number-average fiber diameter and average degree of polymerization dispersed in a dispersion medium such as water.

The deodorants comprise metal-containing oxidized cellulose nanofibers, each strand of which effectively contains a metal in salt form. Thus, the deodorants show superior deodorizing performance.

In particular, metal-containing oxidized cellulose nanofibers containing copper in salt form (copper-containing oxidized cellulose nanofibers) show particularly superior deodorizing performance against hydrogen sulfide.

As described above, the metal-containing oxidized cellulose nanofibers in the deodorant preferably have a number-average fiber length of 50 nm to 2,000 nm, more preferably 70 nm to 1,500 nm, even more preferably 100 nm to 1,000 nm, particularly preferably 400 nm to 600 nm.

The metal-containing oxidized cellulose nanofibers in the deodorant have an average degree of polymerization of 100 to 2,000, preferably 300 to 1,500, more preferably 500 to 1,000, particularly preferably 500 to 700.

EXAMPLES

The following provides a more specific description of the present disclosure based on Examples, which however shall not be construed as limiting. In the following description, "%" used to express quantities are by mass, unless otherwise specified.

In Examples, the carboxyl group amount of oxidized cellulose nanofibers, and the number-average fiber diameter, number-average fiber length, degree of polymerization, metal amount and deodorizing performance of metal-containing oxidized cellulose nanofibers were evaluated using the methods described below.

<Carboxyl Group Amount>

60 mL of a dispersion containing 0.5% to 1% by mass of oxidized cellulose nanofibers was prepared from a pulp sample of oxidized cellulose nanofibers precisely weighed in dry weight. Next, after the pH of the dispersion was adjusted to about 2.5 with 0.1M hydrochloric acid, changes in electrical conductivity were observed until the pH reached 11 by dropwise addition of 0.05M sodium hydroxide aqueous solution. The amount of carboxyl groups in the oxidized cellulose nanofibers was calculated using the following equation based on the volume (V) of sodium hydroxide consumed during the neutralization stage of the weak acid where changes in electrical conductivity are moderate:

$$\text{Carboxyl group amount (mmol/g)} = \{V \text{ (mL)} \times 0.05\} \text{ / mass (g) of pulp sample}$$

<Number-Average Fiber Diameter>

The metal-containing oxidized cellulose nanofiber dispersion was diluted to prepare a dispersion containing 0.0001% by mass of metal-containing oxidized cellulose nanofibers. The resulting dispersion was dropped on mica and dried to form an observation sample. The sample was then observed using an atomic force microscope (Dimension Fast Scan AFM, Bruker; tapping mode), and in an image in which metal-containing oxidized cellulose nanofibers can be confirmed, 5 or more metal-containing oxidized cellulose nanofibers were measured for their fiber diameter and an average value was calculated.

<Number-Average Fiber Length>

The metal-containing oxidized cellulose nanofiber dispersion was diluted to prepare a dispersion containing 0.0001% by mass of metal-containing oxidized cellulose nanofibers. The resulting dispersion was dropped on mica and dried to form an observation sample. The sample was then observed using an atomic force microscope (Dimension Fast Scan AFM, Bruker; tapping mode), and in an image in which metal-containing oxidized cellulose nanofibers can be confirmed, 5 or more metal-containing oxidized cellulose nanofibers were measured for their fiber length and an average value was calculated.

<Degree of Polymerization>

The prepared metal-containing oxidized cellulose nanofibers were reduced with sodium borohydride to reduce remaining aldehyde groups in the molecules to their alcohols. Thereafter, the metal-containing oxidized cellulose nanofibers subjected to the reduction treatment were dissolved in a 0.5M copper ethylenediamine solution and the degree of polymerization was determined by the viscosity method. Specifically, the degree of polymerization was determined in accordance with "Isogai, A., Mutoh, N., Onabe, F., Usuda, M., "Viscosity measurements of cellulose/SO2-amine-dimethylsulfoxide solution", Sen'i Gakkaishi, 45, 299-306 (1989)."

The reduction treatment using sodium borohydride was carried out in order to prevent molecular weight reductions due to the beta elimination reaction that occurs in the process of dissolution into the copper ethylenediamine solution when aldehyde groups remained.

<Metal Amount>

Metals in the metal-containing oxidized cellulose nanofibers were qualified and quantified by ICP-AES. SPS5100 (SII NanoTechnology) was used for the measurement. In addition, the amount of each ion was quantified by ion chromatography. For measurement, DX-500 (DIONEX) was used.

From the measurement results, the amounts of metals forming salts with carboxyl groups of the oxidized cellulose nanofibers were determined.

<Deodorizing Performance>

The prepared aqueous dispersion of metal-containing carboxylated cellulose nanofibers (or aqueous dispersion of TEMPO-oxidized pulp) was uniformly dropped (applied) to one side of filter paper (qualitative filter paper No. 2, 150 mm diameter, ADVANTEC), and the filter paper was placed in an oven at 105°C and dried. The amount of cellulose nanofibers (or TEMPO-oxidized pulp) supported on the filter paper was 2 mg. The filter paper was placed in a sampling bag with a capacity of 5 L (smart bag, PA, 5 L, model AA, GL Sciences Inc.).

The sampling bag was vacuumed using a vacuum pump to remove air, and 1L of nitrogen/ammonia mixed gas with 97 ppm by mass ammonia (for evaluating the deodorizing performance against ammonia), nitrogen/methyl mercaptan mixed gas with 58 ppm by mass methyl mercaptan (for evaluating deodorizing performance against methyl mercaptan), or nitrogen/hydrogen sulfide mixed gas with 54 ppm by mass hydrogen sulfide (for evaluating the deodorizing performance against hydrogen sulfide) was produced. The mixed gas was fed into the sampling bag and the bag was sealed with a rubber cap. The sampling bag was left at room temperature, and the concentration of ammonia, methyl mercaptan or hydrogen sulfide in the sampling bag at 30 minutes, 60 minutes and 180 minutes post gas sealing was measured with a detection tube (Gas Tech, Inc.). Further, in order to confirm the re-emission of the gas during heating in the physical adsorption, the sampling bag was placed in an oven at 40°C for 1 hour, taken out of the oven after 1 hour, and the concentration of ammonia, methyl mercaptan or hydrogen sulfide was measured in ppm by mass.

[Example 1]

(Inventive Example 1)

<Preparation of Dispersion of Oxidized Cellulose Nanofibers>

1 g in dry weight of coniferous bleached kraft pulp, 5 mmol of sodium hypochlorite, 0.1 g (1 mmol) of sodium bromide and 0.016 g (1 mmol) of TEMPO were dispersed in 100 mL of water, stirred gently for 4 hours at room temperature, and washed with distilled water to afford TEMPO-oxidized pulp (oxidized cellulose). The amount of carboxyl groups of the TEMPO-oxidized pulp was 1.4 mmol/g.

Distilled water was then added to the never-dried TEMPO-oxidized pulp to prepare an aqueous dispersion having a solid content concentration of 0.1%. The aqueous dispersion was subjected to defibration treatment for 2 minutes at $7.5 \times 1,000$ rpm using a homogenizer (Physcotron, Microtec Co., Ltd.) and for 4 minutes using an ultrasonic homogenizer (Ultrasonic Generator, Nissei Corporation; V-LEVEL: 4, TIP: 26D) while ice-cooling the surroundings of the container. In this way, an aqueous dispersion containing carboxylated cellulose nanofibers as oxidized cellulose nanofibers was obtained. Thereafter, centrifugation ($\times 12,000$ g ($120 \times 100$ rpm/g), 10 min, 12°C) was performed using a centrifugal separator (M201-1VD, angle rotor: 50F-8AL, SAKUMA) to remove non-defibrated components from the aqueous dispersion of carboxylated cellulose nanofibers to afford a 0.1% clear aqueous dispersion of carboxylated cellulose nanofibers. The carboxylated cellulose nanofibers contained sodium (first metal) in salt form which was derived from the co-oxidizing agent.

<Preparation of Dispersion of Hydrogen-Substituted Oxidized Cellulose Nanofibers>

To 100 mL of the aqueous dispersion of carboxylated cellulose nanofibers was added 1 mL of 1M hydrochloric acid under stirring to adjust the pH to 1. Stirring was continued for 60 minutes (hydrogen substitution step).

Thereafter, the carboxylated cellulose nanofibers gelled by the addition of hydrochloric acid were recovered by centrifugation ($\times 12,000$ g), and the recovered carboxylated cellulose nanofibers were washed with 1M hydrochloric acid and then with a large quantity of distilled water (first washing step).

Next, 100 mL of distilled water was added to afford a 0.1% aqueous dispersion of hydrogen-substituted carboxylated cellulose nanofibers (first dispersing step). 90% or more of the carboxyl groups present on the surface of the hydrogen-substituted carboxylated cellulose nanofibers were substituted by carboxylic acid form as measured by FT-IR (FT/IR-6100, JASCO Corporation) in accordance with Biomacromolecules, 2011, vol.12, pp.518-522.

<Preparation of Dispersion of Metal-Containing Oxidized Cellulose Nanofibers>

50 g of the 0.1% aqueous dispersion of hydrogen-substituted carboxylated cellulose nanofibers was stirred, 18 g of a 0.1% copper (II) acetate aqueous solution was added, and stirring was continued at room temperature for 3 hours (metal substitution step). The carboxylated cellulose nanofibers gelled by the addition of the copper (II) acetate aqueous solution were then recovered by centrifugation ($\times$12,000 g (120$\times$100 rpm/g), 10 min, 12°C) using a centrifugal separator (M201-1VD, angle rotor: 50F-8AL, SAKUMA), and the recovered carboxylated cellulose nanofibers were washed with 0.1% copper (II) acetate aqueous solution and then with a large quantity of distilled water (washing step).

50 ml of distilled water was then added and the dispersion was subjected to ultrasonic treatment for 2 minutes using an ultrasonic homogenizer (Ultrasonic Generator, Nissei Corporation; V-LEVEL: 4, TIP: 26D) while ice-cooling the surroundings of the container to disperse copper-substituted carboxylated cellulose nanofibers. Centrifugation ($\times$12,000 g (120$\times$100 rpm/g), 10 min, 12°C) was performed using a centrifugal separator (M201-1VD, angle rotor: 50F-8AL, SAKUMA) to remove non-defibrated components from the aqueous dispersion of copper-substituted carboxylated cellulose nanofibers to afford a 0.1% clear aqueous dispersion of metal-containing carboxylated cellulose nanofibers (dispersing step).

<Performance Evaluation of Metal-Containing Oxidized Cellulose Nanofibers>

Birefringence was observed when two polarizing plates were arranged in crossed Nicols, light was directed from the opposite side to the viewer, and the aqueous dispersion of metal-containing oxidized cellulose nanofibers was allowed to move in between the polarizing plates. This confirmed that the metal-containing carboxylated cellulose nanofibers were well dispersed in water. From the image by AFM, it was also confirmed that the metal-containing carboxylated cellulose nanofibers had a number-average fiber diameter of 3.13 nm and were dispersed in water at up to the microfibril level. The relationship between birefringence and dispersibility is disclosed in WO2009/069641, for example.

Further, as a result of ICP-AES measurement using SPS5100 (SII NanoTechnology), it was found that copper (Cu) was present in the metal-containing carboxylated cellulose nanofibers at an amount one-half that in moles of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass and the amount of chlorine ions was not greater than 0.1 ppm by mass. These results suggest that sodium ions present in the carboxylated cellulose nanofibers of the metal-containing carboxylated nanofibers are substituted by copper ions and one copper ion is bound per two carboxyl groups.

The metal-containing oxidized cellulose nanofibers had a number-average fiber length of 550 nm and an average degree of polymerization of 600.

Using the resulting aqueous dispersion of metal-containing carboxylated cellulose nanofibers as a spray deodorant, the deodorizing performance against ammonia was evaluated according to the above-described method of evaluating the deodorizing performance.

The evaluation result is shown in Table 1 as Inventive Example 1.

(Inventive Example 2)

<Preparation of Dispersion of Oxidized Cellulose Nanofibers>

In the same manner as in Example 1, a 0.1% aqueous dispersion of carboxylated cellulose nanofibers was obtained.

<Preparation of Dispersion of Hydrogen-Substituted Oxidized Cellulose Nanofibers>

In the same manner as in Example 1, a 0.1% aqueous dispersion of hydrogen-substituted carboxylated cellulose nanofibers was obtained.

<Preparation of Dispersion of Metal-Containing Oxidized Cellulose Nanofibers>

In the metal substation step, 50 g of the 0.1% aqueous dispersion of hydrogen-substituted carboxylated cellulose nanofibers was stirred, 19.5 g of a 0.1% zinc (II) acetate aqueous solution was added, and stirring was continued at room temperature for 3 hours (metal substitution step). The carboxylated cellulose nanofibers gelled by the addition of the zinc (II) acetate aqueous solution were then recovered by centrifugation ($\times$12,000 g (120$\times$100 rpm/g), 10 min, 12°C) using a centrifugal separator (M201-1VD, angle rotor: 50F-8AL, SAKUMA), and the recovered carboxylated cellulose nanofibers were washed with 0.1% zinc (II) acetate aqueous solution and then with a large quantity of distilled water (washing step).

50 ml of distilled water was then added and the dispersion was subjected to ultrasonic treatment for 2 minutes using an ultrasonic homogenizer (Ultrasonic Generator, Nissei Corporation; V-LEVEL: 4, TIP: 26D) while ice-cooling the surroundings of the container to disperse zinc-substituted carboxylated cellulose nanofibers. Centrifugation ($\times$12,000 g (120$\times$100 rpm/g), 10 min, 12°C) was performed using a centrifugal separator (M201-1VD, angle rotor: 50F-8AL, SAKUMA) to remove non-defibrated components from the aqueous dispersion of zinc-substituted carboxylated cellulose nanofibers to afford a 0.1% clear aqueous dispersion of metal-containing carboxylated cellulose nanofibers (dispersing step).

<Performance Evaluation of Metal-Containing Oxidized Cellulose Nanofibers>

Birefringence was observed when two polarizing plates were arranged in crossed Nicols, light was directed from the opposite side to the viewer, and the aqueous dispersion of metal-containing oxidized cellulose nanofibers was allowed to move in between the polarizing plates. This confirmed that the metal-containing carboxylated cellulose nanofibers were well dispersed in water. From the image by AFM, it was also confirmed that the metal-containing carboxylated cellulose nanofibers had a number-average fiber diameter of 3.15 nm and were dispersed in water at up to the microfibril level.

Further, as a result of ICP-AES measurement using SPS5100 (SII NanoTechnology), it was found that zinc (Zn) was present in the metal-containing carboxylated cellulose nanofibers at an amount one-half that in moles of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass and the amount of chlorine ions was not greater than 0.1 ppm by mass. These results suggest that sodium ions present in the carboxylated cellulose nanofibers of the metal-containing carboxylated nanofibers are substituted by zinc ions and one zinc ion is bound per two carboxyl groups.

The metal-containing carboxylated cellulose nanofibers had a number-average fiber length of 550 nm and an average degree of polymerization of 600.

Using the resulting aqueous dispersion of metal-containing carboxylated cellulose nanofibers as a spray deodorant, the deodorizing performance against ammonia was evaluated according to the above-described method of evaluating the deodorizing performance.

The evaluation result is shown in Table 1 as Inventive Example 2.

(Comparative Example 1)

<Preparation of Aqueous Dispersion>

1 g in dry weight of coniferous bleached kraft pulp, 5 mmol of sodium hypochlorite, 0.1 g (1 mmol) of sodium bromide and 0.016 g (1 mmol) of TEMPO were dispersed in 100 mL of water, stirred gently for 4 hours at room temperature, and washed with distilled water to afford a TEMPO-oxidized pulp (oxidized cellulose). The amount of carboxyl groups of the TEMPO-oxidized pulp was 1.4 mmol/g.

Thereafter, distilled water was added to the never-dried TEMPO-oxidized pulp to prepare an aqueous dispersion having a solid content concentration of 0.1%.

<Preparation of Aqueous Dispersion of TEMPO-Oxidized Pulp>

In the metal substitution step, 50 g of the 0.1% aqueous dispersion was stirred, 19.5 g of a 0.1% zinc (II) acetate aqueous solution was added, and stirring was continued at room temperature for 3 hours. Subsequently, the TEMPO-oxidized pulp was recovered by centrifugation ($\times$12,000 g) and washed with 0.1% zinc (II) acetate aqueous solution (washing step).

After washing the recovered TEMPO-oxidized pulp with a large quantity of distilled water, 50 ml of distilled water was added to afford a 0.1% aqueous dispersion of TEMPO-oxidized pulp (dispersing step).

<Performance Evaluation of TEMPO-Oxidized Pulp>

As a result of ICP-AES measurement using SPS5100 (SII NanoTechnology), it was found that zinc (Zn) was present in the resulting TEMPO-oxidized pulp at an amount one-half that in moles of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass. These results suggest that sodium ions of the TEMPO-oxidized pulp are substituted by zinc ions and one zinc ion is bound per two carboxyl groups.

The TEMPO-oxidized pulp had a number-average fiber diameter of 20 μm and a number-average fiber length of 1 mm. Measurement of the average degree of polymerization of the TEMPO-oxidized pulp failed due to re-aggregation.

Using the resulting aqueous dispersion of TEMPO-oxidized pulp as a spray deodorant, the deodorizing performance against ammonia was evaluated according to the above-described method of evaluating the deodorizing performance.

The evaluation result is shown in Table 1 as Comparative Example 1.

(Comparative Example 2)

Without an aqueous dispersion of metal-containing carboxylated cellulose nanofibers, only a filter paper sheet (qualitative filter paper No. 2, 150 mm diameter, ADVANTEC) was put in an oven at 105°C and dried. The filter paper sheet was subjected to the method for evaluating the deodorizing performance against ammonia.

That is, this comparative example is directed to evaluation of the deodorizing performance of the filter paper sheet against ammonia without using an aqueous dispersion of metal-containing carboxylated cellulose nanofibers.

The evaluation result is shown in Table 1 as Comparative Example 2.

(Comparative Example 3)

An empty sampling bag was vacuumed using a vacuum pump to remove air, and 1L of nitrogen/ammonia mixed gas with 97 ppm by mass ammonia was fed into the sampling bag and the bag was sealed with a rubber cap. The sampling bag was left at room temperature, and the concentration of ammonia gas in the sampling bag at 30 minutes, 60 minutes and 180 minutes post gas sealing was measured with a detection tube (Gas Tech, Inc.). The sampling bag was placed in an oven at 40°C for 1 hour, taken out of the oven after 1 hour, and the concentration of ammonia was measured in ppm by mass.

That is, this comparative example is directed to evaluation of the deodorizing performance of the sampling bag itself without using an aqueous dispersion of metal-containing carboxylated cellulose nanofibers.

The evaluation result is shown in Table 1 as Comparative Example 3.

[Table 1]

| Time (min) | 0 | 30 | 60 | 180 | After 1h in 40°C oven |
|---|---|---|---|---|---|
| Inventive Example 1 | 97 | 8 | 8 | 6 | 6 |
| Inventive Example 2 | 97 | 8 | 8 | 7 | 7 |
| Comparative Example 1 | 97 | 15 | 11 | 9 | 9 |
| Comparative Example 2 | 97 | 25 | 21 | 18 | 17 |
| Comparative Example 3 | 97 | 80 | 79 | 68 | 56 |
| *The units of measure are ppm by mass | | | | | |

From Table 1, it can be seen that metal-containing carboxylated cellulose nanofibers used in the disclosed deodorant have a superior deodorizing effect on ammonia.

[Example 2]

(Inventive Example 3)

Using the aqueous dispersion of metal-containing carboxylated cellulose nanofibers obtained in Inventive Example 1 as a spray deodorant, the deodorizing performance against methyl mercaptan was evaluated according to the method for evaluating the deodorizing performance.

The evaluation result is shown in Table 2 as Inventive Example 3.

(Comparative Example 4)

<Preparation of Aqueous Dispersion>

An aqueous dispersion was prepared in the same procedure as in Comparative Example 1 to prepare an aqueous dispersion having a solid content concentration of 0.1%.

<Preparation of Aqueous Dispersion of TEMPO-Oxidized Pulp>

In the metal substitution step, 50 g of the 0.1% aqueous dispersion was stirred, 18 g of a 0.1% copper (II) acetate aqueous solution was added, and stirring was continued at room temperature for 3 hours (metal substitution step). Subsequently, the TEMPO-oxidized pulp was recovered by centrifugation ($\times$12,000 g) and washed with 0.1% copper (II) acetate aqueous solution (washing step).

After washing the recovered TEMPO-oxidized pulp with a large quantity of distilled water, 50 ml of distilled water was added to afford a 0.1% aqueous dispersion of TEMPO-oxidized pulp (dispersing step).

<Performance Evaluation of TEMPO-Oxidized Pulp>

As a result of ICP-AES measurement using SPS5100 (SII NanoTechnology), it was found that copper (Cu) was present in the TEMPO-oxidized pulp at an amount one-half that in moles of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass. These results suggest that sodium ions of the TEMPO-oxidized pulp are substituted by copper ions and one copper ion is bound per two carboxyl groups.

The TEMPO-oxidized pulp had a number-average fiber diameter of 20 $\mu$m and a number-average fiber length of 1 mm. Measurement of the average degree of polymerization of the TEMPO-oxidized pulp failed due to re-aggregation.

Using the resulting aqueous dispersion of TEMPO-oxidized pulp as a spray deodorant, the deodorizing performance against methyl mercaptan was evaluated according to the above-described method of evaluating the deodorizing performance.

The evaluation result is shown in Table 2 as Comparative Example 4.

(Inventive Example 4)

<Preparation of Dispersion of Oxidized Cellulose Nanofibers>

In the same manner as in Inventive Example 1, a 0.1% aqueous dispersion of carboxylated cellulose nanofibers was obtained.

<Preparation of Dispersion of Hydrogen-Substituted Oxidized Cellulose Nanofibers>

In the same manner as in Inventive Example 1, a 0.1% aqueous dispersion of hydrogen-substituted carboxylated cellulose nanofibers was obtained.

<Preparation of Dispersion of Metal-Containing Oxidized Cellulose Nanofibers>

In the metal substitution step, 50 g of the 0.1% aqueous dispersion of hydrogen-substituted carboxylated cellulose nanofibers was stirred, 18 g of a 0.1% silver (I) acetate aqueous solution was added, and stirring was continued at room temperature for 3 hours (metal substitution step). The carboxylated cellulose nanofibers gelled by the addition of the silver (I) acetate aqueous solution were then recovered by centrifugation ($\times$12,000 g (120$\times$100 rpm/g), 10 min, 12°C) using a centrifugal separator (M201-1VD, angle rotor: 50F-8AL, SAKUMA), and the recovered carboxylated cellulose nanofibers were washed with 0.1% silver (I) acetate aqueous solution and then with a large quantity of distilled water (washing step).

50 ml of distilled water was then added and the dispersion was subjected to ultrasonic treatment for 2 minutes using an ultrasonic homogenizer (Ultrasonic Generator, Nissei Corporation; V-LEVEL: 4, TIP: 26D) while ice-cooling the surroundings of the container to disperse silver-substituted carboxylated cellulose nanofibers. Centrifugation ($\times$12,000 g (120$\times$100 rpm/g), 10 min, 12°C) was performed using a centrifugal separator (M201-1VD, angle rotor: 50F-8AL, SAKUMA) to remove non-defibrated components from the aqueous dispersion of silver-substituted carboxylated cellulose nanofibers to afford a 0.1% clear aqueous dispersion of metal-containing carboxylated cellulose nanofibers (dispersing step).

<Performance Evaluation of Metal-Containing Oxidized Cellulose Nanofibers>

Birefringence was observed when two polarizing plates were arranged in crossed Nicols, light was directed from the opposite side to the viewer, and the aqueous dispersion of metal-containing oxidized cellulose nanofibers was allowed to move in between the polarizing plates. This confirmed that the metal-containing carboxylated cellulose nanofibers were well dispersed in water. From the image by AFM, it was also confirmed that the metal-containing carboxylated cellulose nanofibers had a number-average fiber diameter of 3.13 nm and were dispersed in water at up to the microfibril level.

As a result of ICP-AES measurement using SPS5100 (SII NanoTechnology), it was found that silver (Ag) was present in the metal-containing carboxylated cellulose nanofibers at an amount equivalent to that of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass and the amount of chlorine ions was not greater than 0.1 ppm by mass. These results suggest that sodium ions present in the carboxylated cellulose nanofibers of the metal-containing carboxylated nanofibers are substituted by silver ions.

The metal-containing oxidized cellulose nanofibers had a number-average fiber length of 550 nm and an average degree of polymerization of 600.

Using the resulting aqueous dispersion of metal-containing carboxylated cellulose nanofibers as a spray deodorant, the deodorizing performance against methyl mercaptan was evaluated according to the above-described method of evaluating the deodorizing performance.

The evaluation result is shown in Table 2 as Inventive Example 4.

(Comparative Example 5)

<Preparation of Aqueous Dispersion>

An aqueous dispersion was prepared in the same procedure as in Comparative Example 1 to prepare an aqueous dispersion having a solid content concentration of 0.1%.

<Preparation of Aqueous Dispersion of TEMPO-Oxidized Pulp>

In the metal substitution step, 50 g of the 0.1% aqueous dispersion was stirred, 18 g of a 0.1% silver (I) acetate aqueous solution was added, and stirring was continued at room temperature for 3 hours (metal substitution step). Subsequently, the TEMPO-oxidized pulp was recovered by centrifugation ($\times$12,000 g) and washed with 0.1% silver (I) acetate aqueous solution (washing step).

After washing the recovered TEMPO-oxidized pulp with a large quantity of distilled water, 50 ml of distilled water was added to afford a 0.1% aqueous dispersion of TEMPO-oxidized pulp (dispersing step).

<Performance Evaluation of TEMPO-Oxidized Pulp>

As a result of ICP-AES measurement using SPS5100 (SII NanoTechnology), it was found that silver (Ag) was present in the TEMPO-oxidized pulp at an amount equivalent to that of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass. These results confirm that sodium ions of the TEMPO-oxidized pulp are substituted by silver ions.

The TEMPO-oxidized pulp had a number-average fiber diameter of 20 $\mu$m and a number-average fiber length of 1 mm. Measurement of the average degree of polymerization of the TEMPO-oxidized pulp failed due to re-aggregation.

Using the resulting aqueous dispersion of TEMPO-oxidized pulp as a spray deodorant, the deodorizing performance against methyl mercaptan was evaluated according to the above-described method of evaluating the deodorizing performance.

The evaluation result is shown in Table 2 as Comparative Example 5.

(Comparative Example 6)

As Comparative Example 6, the deodorizing performance of a filter paper sheet against methyl mercaptan was evaluated by the same procedure as in Comparative Example 2 without using an aqueous dispersion of metal-containing carboxylated cellulose nanofibers.

The evaluation result is shown in Table 2 as Comparative Example 6.

(Comparative Example 7)

As Comparative Example 7, the deodorizing performance of a sampling bag itself against methyl mercaptan without using an aqueous dispersion of metal-containing carboxylated cellulose nanofibers was evaluated by the same procedure as in Comparative Example 3. The initial concentration of methyl mercaptan in the sampling bag was set to 58 ppm by mass.

The evaluation result is shown in Table 2 as Comparative Example 7.

Table 2

| Time (min) | 0 | 30 | 60 | 180 | After 1h in 40°C oven |
|---|---|---|---|---|---|
| Inventive Example 3 | 58 | 10 | 8 | 8 | 7 |
| Comparative Example 4 | 58 | 49 | 47 | 40 | 35 |
| Inventive Example 4 | 58 | 37 | 31 | 30 | 28 |
| Comparative Example 5 | 58 | 51 | 51 | 51 | 49 |
| Comparative Example 6 | 58 | 58 | 58 | 58 | 55 |
| Comparative Example 7 | 58 | 58 | 58 | 58 | 58 |
| *The units of measure are ppm by mass | | | | | |

From Table 2, it can be seen that metal-containing carboxylated cellulose nanofibers used in the disclosed deodorant have a superior deodorizing effect on methyl mercaptan.

[Example 3]

(Inventive Example 5)

Using the aqueous dispersion of metal-containing carboxylated cellulose nanofibers obtained in Inventive Example 4 as a spray deodorant, the deodorizing performance against hydrogen sulfide was evaluated according to the method for evaluating the deodorizing performance.

The evaluation result is shown in Table 3 as Inventive Example 5.

(Comparative Example 8)

Using the aqueous dispersion of TEMPO-oxidized pulp obtained in Comparative Example 5 as a spray deodorant, the deodorizing performance against hydrogen sulfide was evaluated according to the above-described method of evaluating the deodorizing performance.

The evaluation result is shown in Table 3 as Comparative Example 8.

(Comparative Example 9)

As Comparative Example 9, the deodorizing performance of a filter paper sheet against hydrogen sulfide was evaluated by the same procedure as in Comparative Example 2 without using an aqueous dispersion of metal-containing carboxylated cellulose nanofibers.

The evaluation result is shown in Table 3 as Comparative Example 9.

(Comparative Example 10)

As Comparative Example 10, the deodorizing performance of a sampling bag itself against hydrogen sulfide without using an aqueous dispersion of metal-containing carboxylated cellulose nanofibers was evaluated by the same procedure as in Comparative Example 3. The initial concentration of hydrogen sulfide in the sampling bag was set to 54 ppm by mass.

The evaluation result is shown in Table 3 as Comparative Example 10.

[Table 3]

| Time (min) | 0 | 30 | 60 | 180 | After 1h in 40°C oven |
|---|---|---|---|---|---|
| Inventive Example 5 | 54 | 30 | 28 | 26 | 21 |
| Comparative Example 8 | 54 | 45 | 43 | 42 | 35 |
| Comparative Example 9 | 54 | 52 | 51 | 51 | 50 |
| Comparative Example 10 | 54 | 50 | 50 | 50 | 50 |
| *The units of measure are ppm by mass | | | | | |

From Table 3, it can be seen that metal-containing carboxylated cellulose nanofibers used in the disclosed deodorant have a superior deodorizing effect on hydrogen sulfide.

(Example 4)

(Inventive Example 6)

<Preparation of Dispersion of Oxidized Cellulose Nanofibers>

In the same manner as in Inventive Example 1, a 0.1% aqueous dispersion of carboxylated cellulose nanofibers was obtained.

<Preparation of Dispersion of Metal-Containing Oxidized Cellulose Nanofibers>

50 g of the 0.1% aqueous dispersion of carboxylated cellulose nanofibers was stirred, 18 g of a 0.1% copper (II) acetate aqueous solution was added as a copper salt aqueous solution, and stirring was continued at room temperature for 3 hours (metal substitution step). The carboxylated cellulose nanofibers gelled by the addition of the copper (II) acetate aqueous solution were then recovered by centrifugation ($\times$12,000 g (120$\times$100 rpm/g), 10 min, 12°C) using a centrifugal separator (M201-1VD, angle rotor: 50F-8AL, SAKUMA), and the recovered carboxylated cellulose nanofibers were washed with 0.1% copper (II) acetate aqueous solution and then with a large quantity of distilled water (washing step).
50 ml of distilled water was then added and the dispersion was subjected to ultrasonic treatment for 2 minutes using an ultrasonic homogenizer (Ultrasonic Generator, Nissei Corporation; V-LEVEL: 4, TIP: 26D) while ice-cooling the surroundings of the container to disperse copper-substituted carboxylated cellulose nanofibers. Centrifugation ($\times$12,000 g (120$\times$100 rpm/g), 10 min, 12°C) was performed using a centrifugal separator (M201-1VD, angle rotor: 50F-8AL, SAKUMA) to remove non-defibrated components from the aqueous dispersion of copper-substituted carboxylated cellulose nanofibers to afford a 0.1% clear aqueous dispersion of metal-containing carboxylated cellulose nanofibers (dispersing step).

<Performance Evaluation of Metal-Containing Oxidized Cellulose Nanofibers>

Birefringence was observed when two polarizing plates were arranged in crossed Nicols, light was directed from the opposite side to the viewer, and the aqueous dispersion of metal-containing oxidized cellulose nanofibers was allowed to move in between the polarizing plates. This confirmed that the metal-containing carboxylated cellulose nanofibers were well dispersed in water. From the image by AFM, it was also confirmed that the metal-containing carboxylated cellulose nanofibers had a number-average fiber diameter of 3.13 nm and were dispersed in water at up to the microfibril level. The relationship between birefringence and dispersibility is disclosed in WO2009/069641, for example.
Further, as a result of ICP-AES measurement using SPS5100 (SII NanoTechnology), it was found that copper (Cu) was present in the metal-containing carboxylated cellulose nanofibers at an amount one-half that in moles of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass. These results suggest that sodium ions present in the carboxylated cellulose nanofibers of the metal-containing carboxylated nanofibers are substituted by copper ions and one copper ion is bound per two carboxyl groups.
The metal-containing oxidized cellulose nanofibers had a number-average fiber length of 550 nm and an average degree of polymerization of 600.

Using the resulting aqueous dispersion of metal-containing carboxylated cellulose nanofibers as a spray deodorant, the deodorizing performance against hydrogen sulfide was evaluated according to the above-described method of evaluating the deodorizing performance. The evaluation result is shown in Table 4 as Inventive Example 6.

(Comparative Example 11)

<Preparation of Dispersion of Oxidized Cellulose Nanofibers>

An aqueous dispersion was prepared in the same procedure as in Inventive Example 6 to prepare an aqueous dispersion having a solid content concentration of 0.1%.

<Preparation of Aqueous Dispersion of Metal-Containing Oxidized Cellulose Nanofibers>

In the metal substitution step, 50 g of the 0.1% aqueous dispersion was stirred, 18 g of a 0.1% copper (II) acetate aqueous solution was added, and stirring was continued at room temperature for 3 hours (metal substitution step). The gelled metal-containing carboxylated cellulose nanofibers were then recovered by centrifugation ($\times$12,000 g) and washed with 0.1% copper (II) acetate aqueous solution (washing step).

After washing the recovered gelled metal-containing carboxylated cellulose nanofibers with a large quantity of distilled water, 50 ml of distilled water was added to afford a 0.1% aqueous dispersion of metal-containing carboxylated cellulose nanofibers (dispersing step).

<Performance Evaluation of Gelled Metal-Containing Carboxylated Cellulose Nanofibers>

As a result of ICP-AES measurement using SPS5100 (SII NanoTechnology), it was found that copper (Cu) was present in the gelled metal-containing carboxylated cellulose nanofibers at an amount one-half that in moles of the carboxyl groups of the carboxylated cellulose nanofibers, and the amount of sodium was not greater than 1 ppm by mass. Further, as a result of quantitation of the ion amount by ion chromatography using DX-500 (DIONEX), it was found that the amount of acetate ions was not greater than 0.5 ppm by mass. These results suggest that sodium ions present in the gelled metal-containing carboxylated cellulose nanofibers are substituted by copper ions and one copper ion is bound per two carboxyl groups.

The gelled metal-containing carboxylated cellulose nanofibers had a number-average fiber diameter of 20 $\mu$m and a number-average fiber length of 1 mm. Measurement of the average degree of polymerization of the gelled metal-containing carboxylated cellulose nanofibers failed due to re-aggregation.

Using the aqueous dispersion of gelled metal-containing carboxylated cellulose nanofibers as a spray deodorant, the deodorizing performance against hydrogen sulfide was evaluated according to the method for evaluating the deodorizing performance. The evaluation result is shown in Table 4 as Comparative Example 11.

(Comparative Example 12)

As Comparative Example 12, the deodorizing performance of a filter paper sheet against hydrogen sulfide was evaluated by the same procedure as in Comparative Example 2 without using an aqueous dispersion of metal-containing carboxylated cellulose nanofibers.

The evaluation result is shown in Table 4 as Comparative Example 12.

(Comparative Example 13)

As Comparative Example 13, the deodorizing performance of a sampling bag itself against hydrogen sulfide without using an aqueous dispersion of metal-containing carboxylated cellulose nanofibers was evaluated by the same procedure as in Comparative Example 3. The initial concentration of hydrogen sulfide in the sampling bag was set to 54 ppm by mass.

The evaluation result is shown in Table 4 as Comparative Example 13.

[Table 4]

| Time (min) | 0 | 30 | 60 | 180 | After 1h in 40°C oven |
|---|---|---|---|---|---|
| Inventive Example 6 | 54 | 18 | 14 | 6 | <2.5 |
| Comparative Example 11 | 54 | 40 | 37 | 30 | 7 |

(continued)

| Time (min) | 0 | 30 | 60 | 180 | After 1h in 40°C oven |
|---|---|---|---|---|---|
| Comparative Example 12 | 54 | 52 | 51 | 51 | 50 |
| Comparative Example 13 | 54 | 50 | 50 | 50 | 50 |
| *The units of measure are ppm by mass | | | | | |

From Table 4, it can be seen that metal-containing carboxylated cellulose nanofibers used in the disclosed deodorant have a superior deodorizing effect on hydrogen sulfide.

INDUSTRIAL APPLICABILITY

According to the present disclosure, it is possible to provide a deodorant having superior deodorizing properties.

**Claims**

1.    A deodorant comprising:

metal-containing oxidized cellulose nanofibers containing a metal other than sodium in salt form and having a number-average fiber diameter of 100 nm or less, wherein
the metal-containing oxidized cellulose nanofibers have an average degree of polymerization of 100 to 2,000, wherein the metal other than sodium is at least one metal selected from the group consisting of copper, zinc, and silver,
wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and
wherein the metal-containing oxidized cellulose nanofibers are metal-containing carboxylated cellulose nanofibers.

2.    The deodorant of claim 1, wherein the metal-containing oxidized cellulose nanofibers have a number-average fiber length of 50 nm to 2,000 nm.

3.    The deodorant of claim 1 or 2, further comprising a dispersion medium, wherein
the metal-containing oxidized cellulose nanofibers are dispersed in the dispersion medium.

4.    The deodorant of claim 3, wherein the dispersion medium is water.

5.    A method of producing a deodorant, comprising:

contacting oxidized cellulose nanofibers containing a first metal in salt form, dispersed in a solvent, with a salt of a second metal other than the first metal to produce metal-containing oxidized cellulose nanofibers containing the second metal in salt form and having a number-average fiber diameter of 100 nm or less, wherein
the metal-containing oxidized cellulose nanofibers have an average degree of polymerization of 100 to 2,000, wherein the first metal is sodium and the second metal is at least one metal selected from the group consisting of copper, zinc, and silver,
wherein the average degree of polymerization is measured as with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and
wherein the oxidized cellulose nanofibers are carboxylated cellulose nanofibers.

6.    A method of producing a deodorant, comprising:

contacting oxidized cellulose nanofibers containing a first metal in salt form, dispersed in a solvent, with a strong acid to substitute ions of the first metal contained in salt form by hydrogen atoms; and

contacting the oxidized cellulose nanofibers in which the ions of the first metal have been substituted by hydrogen atoms, dispersed in a solvent, with a salt of a second metal other than the first metal to produce metal-containing oxidized cellulose nanofibers containing the second metal in salt form and having a number-average fiber diameter of 100 nm or less, wherein

the metal-containing oxidized cellulose nanofibers have an average degree of polymerization of 100 to 2,000, and wherein the first metal is sodium and the second metal is at least one metal selected from the group consisting of copper, zinc, and silver,

wherein the average degree of polymerization is measured with the following method: the prepared metal-containing oxidized cellulose nanofibers were reduced with sodium borohydride to reduce remaining aldehyde groups in the molecules to their alcohols; thereafter, the metal-containing oxidized cellulose nanofibers subjected to the reduction treatment were dissolved in a 0.5M copper ethylenediamine solution and the degree of polymerization was determined by the viscosity method, and

wherein the oxidized cellulose nanofibers are carboxylated cellulose nanofibers.

7. The method of producing a deodorant of claim 5 or 6, wherein the metal-containing oxidized cellulose nanofibers have a number-average fiber length of 50 nm to 2,000 nm.

8. The method of producing a deodorant of any one of claims 5 to 7, further comprising dispersing the metal-containing oxidized cellulose nanofibers in a dispersion medium.

9. The method of producing a deodorant of claim 8, wherein the dispersion medium is water.

**Patentansprüche**

1. Deodorant umfassend:

Metall-enthaltende oxidierte Cellulosenanofasern enthaltend ein Metall außer Natrium in Salzform und mit einem zahlenmittleren Faserdurchmesser von 100 nm oder weniger, wobei

die Metall-enthaltenden oxidierten Cellulosenanofasern einen durchschnittlichen Polymerisationsgrad von 10 bis 2000 aufweisen,

wobei das Metall außer Natrium mindestens ein Metall ausgewählt aus der Gruppe bestehend aus Kupfer, Zink, und Silber ist,

wobei der durchschnittliche Polymerisationsgrad mit dem folgenden Verfahren gemessen wird: die hergestellten Metall-enthaltenden oxidierten Cellulosenanofasern werden mit Natriumborhydrid reduziert, um verbleibende Aldehydgruppen in den Molekülen zu deren Alkoholen zu reduzieren; danach werden die Metall-enthaltenden oxidierten Cellulosenanofasern, die der Reduktionsbehandlung unterzogen wurden, in einer 0.5 M Kupferethylendiaminlösung aufgelöst und der Polymerisationsgrad wurde mittels des Viskositätsverfahrens bestimmt, und

wobei die Metall-enthaltenden oxidierten Cellulosenanofasern Metall-enthaltende carboxylierte Cellulosenanofasern sind.

2. Deodorant nach Anspruch 1, wobei die Metall-enthaltenden oxidierten Cellulosenanofasern eine zahlenmittlere Faserlänge von 50 nm bis 2000 nm aufweisen.

3. Deodorant nach Anspruch 1 oder 2 ferner umfassend ein Dispersionsmedium, wobei die Metall-enthaltenden oxidierten Cellulosenanofasern in dem Dispersionsmedium dispergiert sind.

4. Deodorant nach Anspruch 3, wobei das Dispersionsmedium Wasser ist.

5. Verfahren zur Herstellung eines Deodorants umfassend:

Kontaktieren oxidierter Cellulosenanofasern enthaltend ein erstes Metall in Salzform, welche in einem Lösemittel dispergiert sind, mit einem Salz eines zweiten Metalls außer dem ersten Metall, um Metall-enthaltende oxidierte Cellulosenanofasern enthaltend das zweite Metall in Salzform und mit einem zahlenmittleren Faserdurchmesser von 100 nm oder weniger herzustellen, wobei

die Metall-enthaltenden oxidierten Cellulosenanofasern einen durchschnittlichen Polymerisationsgrad von 100 bis 2000 aufweisen,

wobei das erste Metall Natrium ist und das zweite Metall mindestens ein Metall ausgewählt aus der Gruppe bestehend aus Kupfer, Zink, und Silber, ist,

wobei der durchschnittliche Polymerisationsgrad mit dem folgenden Verfahren gemessen wird: die hergestellten Metall-enthaltenden oxidierten Cellulosenanofasern werden mit Natriumborhydrid reduziert, um verbleibende Aldehydgruppen in den Molekülen zu deren Alkoholen zu reduzieren; danach werden die Metall-enthaltenden oxidierten Cellulosenanofasern, die der Reduktionsbehandlung unterzogen wurden, in einer 0.5 M Kupferethylendiaminlösung aufgelöst und der Polymerisationsgrad wurde mittels des Viskositätsverfahrens bestimmt, und wobei die oxidierten Cellulosenanofasern carboxylierte Cellulosenanofasern sind.

6. Verfahren zur Herstellung eines Deodorants umfassend:

Kontaktieren oxidierter Cellulosenanofasern enthaltend ein erstes Metall in Salzform, welche in einem Lösemittel dispergiert sind, mit einer starken Säure, um Ionen des ersten Metalls, die in Salzform enthalten sind, durch Wasserstoffatome zu ersetzen; und

Kontaktieren oxidierter Cellulosenanofasern, in welchen die Ionen des ersten Metalls mit Wasserstoffatomen ersetzt wurden, welche in einem Lösemittel dispergiert sind, mit einem Salz eines zweiten Metalls außer dem ersten Metall, um Metall-enthaltende Cellulosenanofasern enthaltend das zweite Metall in Salzform und mit einem zahlenmittleren Durchmesser von 100 nm oder weniger herzustellen, wobei

die Metall-enthaltenden oxidierten Cellulosenanofasern einen durchschnittlichen Polymerisationsgrad von 10 bis 2000 aufweisen, und

wobei das erste Metall Natrium ist und das zweite Metall mindestens ein Metall ausgewählt aus der Gruppe bestehend aus Kupfer, Zink und Silber ist,

wobei der durchschnittliche Polymerisationsgrad mit dem folgenden Verfahren gemessen wird: die hergestellten Metall-enthaltenden oxidierten Cellulosenanofasern werden mit Natriumborhydrid reduziert, um verbleibende Aldehydgruppen in den Molekülen zu deren Alkoholen zu reduzieren; danach werden die Metall-enthaltenden oxidierten Cellulosenanofasern, die der Reduktionsbehandlung unterzogen wurden, in einer 0.5 M Kupferethylendiaminlösung aufgelöst und der Polymerisationsgrad wurde mittels des Viskositätsverfahrens bestimmt, und wobei die oxidierten Cellulosenanofasern carboxylierte Cellulosenanofasern sind.

7. Verfahren zur Herstellung eines Deodorants nach Anspruch 5 oder 6, wobei die Metall-enthaltenden oxidierten Cellulosenanofasern eine zahlenmittlere Faserlänge von 50 nm bis 2000 nm aufweisen.

8. Verfahren zur Herstellung eines Deodorants nach einem der Ansprüche 5 bis 7, ferner umfassend Dispergieren der Metall-enthaltenden oxidierten Cellulosenanofasern in einem Dispersionsmedium.

9. Verfahren zur Herstellung eines Deodorants nach Anspruch 8, wobei das Dispersionsmedium Wasser ist.

**Revendications**

1. Déodorant comportant :

des nanofibres de cellulose oxydée contenant du métal, contenant un métal autre que du sodium sous forme de sel et ayant un diamètre moyen en nombre de fibres de 100 nm ou moins,

dans lequel les nanofibres de cellulose oxydée contenant du métal ont un degré de polymérisation moyen de 100 à 2 000,

dans lequel le métal autre que le sodium est au moins un métal choisi parmi le groupe constitué du cuivre, du zinc et de l'argent,

dans lequel le degré de polymérisation moyen est mesuré par le procédé suivant : les nanofibres de cellulose oxydée contenant du métal préparées ont été réduites avec du borohydrure de sodium pour réduire les groupes aldéhydes restants dans les molécules en leurs alcools, les nanofibres de cellulose oxydée contenant du métal soumises au traitement de réduction ont été ensuite dissoutes dans une solution à 0,5 M d'éthylènediamine de cuivre et le degré de polymérisation a été déterminé par la méthode de viscosité, et

dans lequel les nanofibres de cellulose oxydée contenant du métal sont des nanofibres de cellulose carboxylée contenant du métal.

2. Déodorant selon la revendication 1, dans lequel les nanofibres de cellulose oxydée contenant du métal ont une longueur moyenne en nombre de fibres de 50 nm à 2 000 nm.

3. Déodorant selon la revendication 1 ou 2, comportant en outre un milieu de dispersion, dans lequel les nanofibres de cellulose oxydée contenant du métal sont dispersées dans le milieu de dispersion.

4. Déodorant selon la revendication 3, dans lequel le milieu de dispersion est l'eau.

5. Procédé de production d'un déodorant, comportant les étapes consistant à :

mettre en contact des nanofibres de cellulose oxydée contenant un premier métal sous forme de sel, dispersées dans un solvant, avec un sel d'un second métal autre que le premier métal pour produire des nanofibres de cellulose oxydée contenant du métal contenant le second métal sous forme de sel et ayant un diamètre moyen en nombre de fibres de 100 nm ou moins,
dans lequel les nanofibres de cellulose oxydée contenant du métal ont un degré de polymérisation moyen de 100 à 2 000,
dans lequel le premier métal est le sodium et le second métal est au moins un métal choisi parmi le groupe constitué du cuivre, du zinc et de l'argent,
dans lequel le degré de polymérisation moyen est mesuré par le procédé suivant : les nanofibres de cellulose oxydée contenant du métal préparées ont été réduites avec du borohydrure de sodium pour réduire les groupes aldéhydes restants dans les molécules en leurs alcools, les nanofibres de cellulose oxydée contenant du métal soumises au traitement de réduction ont été ensuite dissoutes dans une solution à 0,5 M d'éthylènediamine de cuivre et le degré de polymérisation a été déterminé par la méthode de viscosité, et
dans lequel les nanofibres de cellulose oxydée sont des nanofibres de cellulose carboxylée.

6. Procédé de production d'un déodorant, comportant les étapes consistant à :

mettre en contact des nanofibres de cellulose oxydée contenant un premier métal sous forme de sel, dispersées dans un solvant, avec un acide fort pour substituer les ions du premier métal contenu sous forme de sel par des atomes d'hydrogène, et
mettre en contact les nanofibres de cellulose oxydée dans lesquelles les ions du premier métal ont été substitués par des atomes d'hydrogène, dispersées dans un solvant, avec un sel d'un second métal autre que le premier métal pour produire des nanofibres de cellulose oxydée contenant du métal contenant le second métal sous forme de sel et ayant un diamètre moyen en nombre de fibres de 100 nm ou moins,
dans lequel les nanofibres de cellulose oxydée contenant du métal ont un degré de polymérisation moyen de 100 à 2 000, et
dans lequel le premier métal est le sodium et le second métal est au moins un métal choisi parmi le groupe constitué du cuivre, du zinc et de l'argent,
dans lequel le degré de polymérisation moyen est mesuré par le procédé suivant : les nanofibres de cellulose oxydée contenant du métal préparées ont été réduites avec du borohydrure de sodium pour réduire les groupes aldéhydes restants dans les molécules en leurs alcools, les nanofibres de cellulose oxydée contenant du métal soumises au traitement de réduction ont été ensuite dissoutes dans une solution à 0,5 M d'éthylènediamine de cuivre et le degré de polymérisation a été déterminé par la méthode de viscosité, et
dans lequel les nanofibres de cellulose oxydée sont des nanofibres de cellulose carboxylée.

7. Procédé de production d'un déodorant selon la revendication 5 ou 6, dans lequel les nanofibres de cellulose oxydée contenant du métal ont une longueur moyenne en nombre de fibres de 50 nm à 2 000 nm.

8. Procédé de production d'un déodorant selon l'une quelconque des revendications 5 à 7, comportant en outre la dispersion des nanofibres de cellulose oxydée contenant du métal dans un milieu de dispersion

9. Procédé de production d'un déodorant selon la revendication 8, dans lequel le milieu de dispersion est l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002371462 A
- JP 2014070158 A
- EP 2395027 A1
- WO 2011074301 A
- WO 2009069641 A

### Non-patent literature cited in the description

- **ISOGAI, A. ; MUTOH, N. ; ONABE, F. ; USUDA, M.** Viscosity measurements of cellulose/SO2-amine-dimethylsulfoxide solution. *Sen'i Gakkaishi,* 1989, vol. 45, 299-306
- *Biomacromolecules,* 2011, vol. 12, 518-522